# EUROPEAN PATENT APPLICATION

(11) **EP 3 991 768 A1**
(43) Date of publication of application: **04.05.2022**
(21) Application number: 20204398.0
(22) Date of filing: 28.10.2020
(51) Int. Cl.: A61M 1/16, A61J 1/14, A61J 1/20, B01F 5/04

(54) **COAXIAL VENTURI SYSTEM**

(71) Applicant: Bellco S.r.l. con Unico Socio, 41037 Modena (IT)
(72) Inventor: VISCIANO, Angela, 41037 Modena (IT); ORLANDINI, Salvatore, 41037 Modena (IT)
(74) Representative: Maschio, Antonio

(57) **Abstract**

A system (10) for re-constituting a solution comprising one or more solutes in a fluid, said system comprising a bag (11) containing one or more solutes in concentrated form, a nozzle (12) for inserting the fluid into the bag, and an outlet (15),
wherein:-
said outlet comprises a ring-shaped one-way valve (16) allowing fluid to leave the bag only,
wherein said nozzle is configured to fit inside the outlet in a coaxial position along the longitudinal axis (L), forming a fitment,
wherein the fitment is configured to fit or attach to an opening (14) in the bag such that the nozzle is positioned to direct fluids into the bag and the one-way valve of the outlet is positioned to direct fluids out of the bag;
wherein said nozzle comprises an internal diameter which reduces along the length of the nozzle, and said nozzle comprises at least one lateral slot or hole (30) at or around the narrowest part of the nozzle, and wherein the internal diameter of said nozzle widens distal to the at least one lateral slot or hole (30) in order to create a venturi effect in fluid flowing through the nozzle;

## Description

### FIELD

The present invention relates to bags and nozzles and valves for use in dissolving various concentrated solutes to form specific solutions. Said solutions can be used in various ways, especially in medical products and systems. Preferably, the systems and apparatus described herein are used in forms of dialysis. In particular, the present invention may be used in medical products for home use.

### BACKGROUND

Many constituents of solutions can be stored as concentrated solutes, such as dry powders or gels, rather than as liquids requiring a large amount of storage space. Re-constitution of the solution using a liquid, usually water, can be carried out when needed. This can be useful in the medical field where storage space or transportation space is restricted. In particular, solutions for use in medical treatments may be re-constituted at home, to prevent the need for treatment to be carried out in a medical centre or hospital. This may be advantageous for many patients who must undergo time consuming and/or frequent medical treatments, such as dialysis patients.

Examples of bags of containers used to re-constitute medical solutions can be seen in US20130333795A1, US2018302711 A1 and US10,022,297B2.

Re-constituting solutions before use can be difficult. Solutes may have poor properties for re-constitution. For example they may be hydroscopic, heavy and sticky, leading to the formation of large particles and clumps when reconstitution is attempted. These problems are particularly relevant in the medical field wherein the properties of a solution may be critical to the health of a patient. Due to at least some of the above described problems, simply adding a liquid to a container of dried constituents may not re-constitute the desired solution correctly. To address this problem, known solutions include using containers with partitions for the different dried constituents, such as those of WO11073274A1, and agitation of the solutions as shown in EP2900295A1 and WO14052596A1. Various types of nozzle and tubing may also be used in the connection of such systems.

However, such products are not ideal as they may be complex to produce and use, and the likelihood of failure or incorrect use is high.

In addition, once a solution is re-constituted, it needs to be emptied, while preventing contamination and leakage.

In view of the above, there is clearly a need for improved systems to re-constitute solutions and then disperse or connect said re-constituted solutions, especially in the medical field.

### SUMMARY

The present invention comprises a system for re-constituting a solution, said system comprising at least a nozzle, an outlet, and a bag. The nozzle is a Venturi nozzle.

The outlet is positioned coaxially to the nozzle along the longitudinal axis, that is said outlet surrounds the nozzle in a concentric arrangement to create a single fitment. The at least one outlet comprises a one-way valve allowing fluid to leave the bag only. In use, the fitment comprising the nozzle and the outlet are positioned at the lowest point of the bag. The outlet is fluidly connectable to a flow path, and in preferred embodiments this is a dialysis flow path.

The bag of the system described herein contains one or more solutes, preferably in a concentrated form. These concentrated solutes may for example be in the form of powder, pellets, super concentrated liquid and/or gel. The bag may also contain peritoneal dialysis fluid (PDF).

The nozzle is configured to be suitable for inserting fluid into the bag.

The outlet is configured to be suitable for removing fluid from the bag. The outlet is preferably configured as a coaxial fitment, which can attach around the nozzle to form an inlet/outlet system. In one embodiment, the coaxial fitment may be configured to comprise a coaxial connector, which interfaces directly with the fluid bag through an integral fitment and is fitted around the venturi nozzle. In another embodiment, the outlet comprises a coaxial fitment, which interfaces with the fluid bag and attaches the coaxial connector to the venturi nozzle. The coaxial fitment may comprise a coaxial tube, which connects the fitment to the connector.

The coaxial connector may be connected to a separate tube to allow fluid to be removed from the bag. Alternatively, the connector may function through the same tube as the venturi nozzle, such that the same tube is used to fill and empty the fluid bag.

The internal diameter of the venturi nozzle reduces along the length of the venturi nozzle in order to create the venturi effect in fluid flowing through the venturi nozzle.

The venturi nozzle comprises at least one lateral slot or hole at or around the point at which the internal diameter is the narrowest.

The fitment comprising the coaxial outlet surrounding the venturi nozzle is configured to fit or attach to an opening in the bag, forming an inlet for a fluid into the bag.

The venturi nozzle and the outlet may comprise separate elements which fit or attach together in a coaxial arrangement, or the fitment may be made as one piece comprising the venturi nozzle and coaxial outlet around it.

In preferred embodiments, the proximal end of the venturi nozzle is configured to attach to a pipe or tube for transporting a fluid to the venturi nozzle.

In one embodiment, the venturi nozzle of the system described herein has an internal diameter which reduces in a gradient along the length of the venturi nozzle.

In one embodiment, the venturi nozzle of the system described herein has an internal diameter reduces in a step-wise manner along the length of the venturi nozzle. In this embodiment, the venturi nozzle preferably comprises at least, two, at least three, at least four, at least five or at least six or more sections, each with a reduced internal diameter compared to the preceding section.

The bag of the system described herein can be any shape. Preferred shapes include rectangular, square, cubic, oval or circular. The bag may be made simply from two sections pf any material sealed together at the edges, or it may be of a more complex formation of multiple sections of material connected by gussets of material.

In preferred embodiment of the system described herein, the bag comprises a V or cone shaped end or section, wherein the openings for the venturi nozzle attachment and the at least one outlet are at the point of the V or cone. The v-shaped end is the bottom of the bag

The bag of the system described herein may be made of any material, preferably a polymer, most preferably a plastic. Examples of materials include polypropylene (PP), PVC and EVA. The bag may be rigid or flexible. Preferably the bag is stretchable, most preferably in any direction. The bag of the system may comprise multiple layers, and may comprise layered films, preferably plastic films, for example PA-PP and PA-PE.

The venturi effect created in the fluid flowing into the bag through the venturi nozzle described herein improves the mixing of the fluid and the one or more solutes. In preferred embodiments the venturi effect increases the fluid flow speed going through the venturi nozzle by at least 5%, at least 10%, at least 20%, or at least 50%. The change in fluid flow speed can be calculated by comparing the flow speed at the entrance to the venturi nozzle with the flow speed where the fluid exits the venturi nozzle.

In preferred embodiments, the use of the venturi nozzle as described herein reduces dissolution time of the solutes compared to the dissolution time taken if the same volume of fluid is added at the same flow rate to a bag of the solutes, at the same temperature and pressure, without the use of the venturi nozzle of the invention. Preferably dissolution time is decreased by at least 5%, at least 10%, at least 20%, at least 50% or at least 75%. Preferably dissolution time is decreased by at least 1 minute, at least 5 minutes, at least 10 minutes, at least 15 minutes or at least 30 minutes, compared to the dissolution time of the same solutes, at the same temperature and pressure, in the same volume of fluid added to a bag at the same flow rate without the venturi nozzle as described herein.

In preferred embodiments, the one or more solutes in the bag are reconstituted into a solution for dialysis, such as peritoneal dialysis, hemodialysis, hemofiltration, hemodiafiltration, ultrafiltration, continuous renal replacement therapy (CRRT), sustained low efficiency dialysis (SLED), continuous venovenous hemofiltration (CVVH), renal replacement therapy (RRT), or slow continuous ultrafiltration (SCUF), or any acute dialysis therapy.

In preferred embodiments, the one or more solutes are reconstituted into a dialysate.

The invention described herein may further comprise a water purification unit configured to be fluidly connectable to the venturi nozzle. The water purification unit may purify water taken from any source, such as mains water from a domestic tap or faucet.

In preferred embodiment, the system described herein comprises a bag with one or more openings fluidly connectable to a dialysis flow path.

The system described herein may be used in any context, but it is envisaged that it will be most useful in the medical field, and specifically in dialysis.

Also described herein is a kit for use in dialysis, said kit comprising:-
a venturi nozzle wherein the internal diameter of the venturi nozzle reduces along the length of the venturi nozzle in order to create the venturi effect in fluid flowing through the venturi nozzle;
at least one outlet comprising a one-way valve;
at least one bag containing one or more solutes which can be dissolved in a fluid, said bag comprising at least one opening;
wherein the venturi nozzle is configured to fit or attach centrally to the outlet forming a coaxial fitment, and wherein said fitment is configured to attach to the bag at the at least one opening.

The kit of the described invention may further comprise a water purification unit. The kits may also include pipes, tubing or other connectors.

Also described herein are methods of re-constituting a solution, preferably at least comprising the steps of:-
(i) filling a bag comprising solute constituents of the solution with fluid passing through a venturi nozzle, wherein the internal diameter of said venturi nozzle reduces along the length of the venturi nozzle in order to create the venturi effect in fluid flowing through the venturi nozzle; and
(ii) allowing the liquid contents of the bag to leave the bag via an outlet comprising a one-way valve, said outlet being positioned coaxially to the venturi nozzle along the longitudinal axis (L).

The advantages of present invention include it enables reduced storage, both in volume and in weight, for solutions. This also aids transport of solutions, as light bags and solutes only need to be transported.

The present invention enables solutions to be created or reconstituted where they are needed, such as in a home. This is advantageous in situations wherein medical treatment may be carried out outside a hospital or medical centre. In particular, it is envisaged the invention described herein is used in creating reconstituting solutions for home dialysis. The invention may enable the production of solutions with highly accurate concentrations of solutes, avoid problems of clumping and partial dissolving of solutes, and allows said solutions to be directed into a home dialysis system with reduced difficulty and possibility of contamination and spillage.

### Brief description of the figures

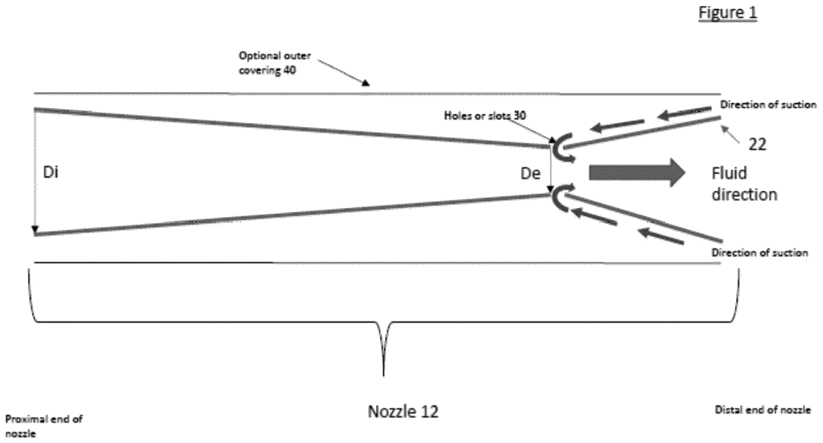

**Figure 1** shows the cross section of a venturi nozzle of the system described herein, wherein the internal diameter reduced in a gradient along the length of the venturi nozzle. The initial diameter is shown as "Di". The narrowest point of the internal diameter of the venturi nozzle is shown as "De". The direction of the negative pressure created by the venturi effect is shown by arrows.

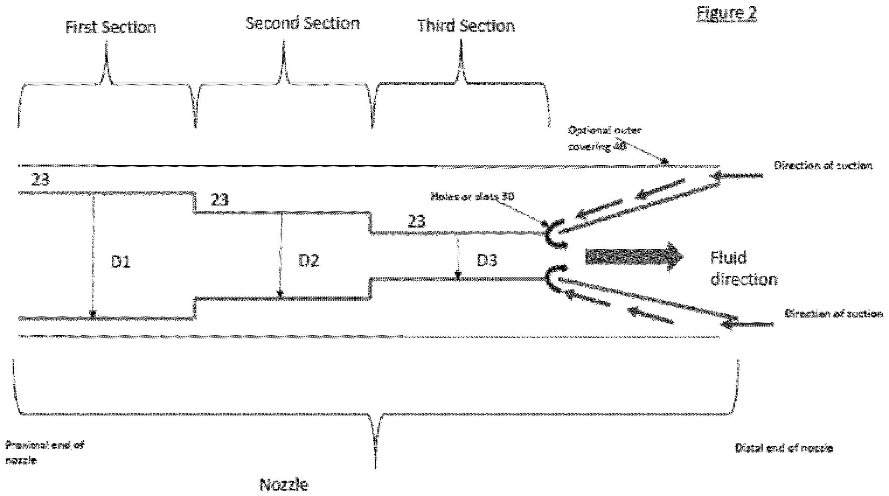

**Figure 2** shows the cross section of a venturi nozzle of the system described herein, wherein the venturi nozzle internal diameter reduces in a step-wise manner along the length of the venturi nozzle. The illustrated embodiment comprises three sections, each with a reduced internal diameter (D1, D2, and D3) compared to the preceding section. The direction of the negative pressure created by the venturi effect is shown by arrows.

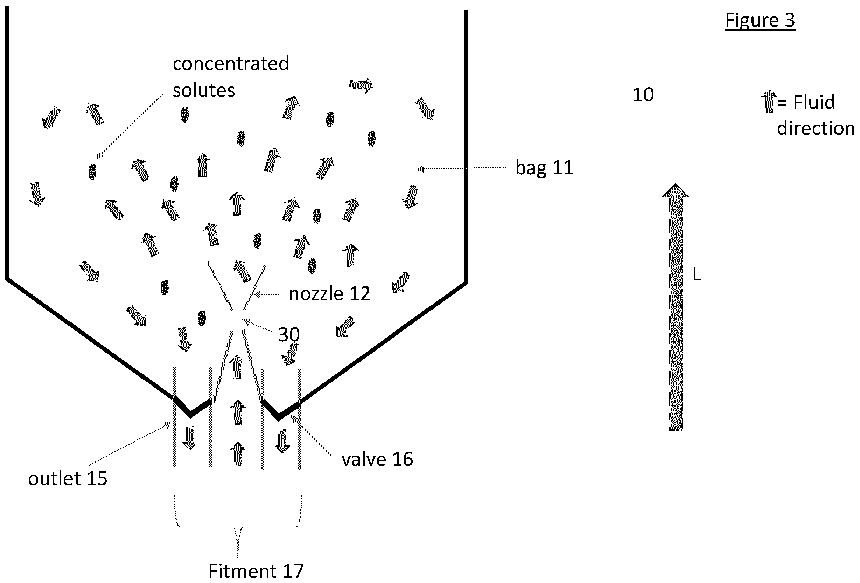

**Figure 3** is a cross-section diagrammatic representation of an embodiment of the system of invention. The bag has a cone shaped section containing one opening (cut through in cross-section at the bottom of the diagram). A fitment comprising a venturi nozzle with a coaxial outlet surrounding it fits into the opening. Fluid travels through the venturi nozzle wherein it undergoes with venturi effect. The negative pressure caused by the narrowing of the venturi nozzle draws air and/or concentrated solutes into the venturi nozzle through the one or more holes in the venturi nozzle (two holes being shown in Figure 3). This causes the fluid to mix with at least a portion of the solutes and then enter the bag (when leaving the venturi nozzle) in a spray, as shown by the arrows. The concentrated solutes remaining inside the bag are disrupted by the spray. The spray is in small drops which allow improved mixing, thus re-constituting the solution inside the bag at the correct concentration, without leaving clumps of solutes undissolved. The reconstituted solution falls to the bottom of the bag due to gravity. The solution leaves the bag through the ring-shaped outlet which is concentric to the nozzle. The one-way valves prevent backflow of the solution into the bag. The bag shown is open across the top, but such an opening is not necessary for the function of the system. The bag may be entirely sealed apart from the single opening for the fitment, or it may comprise one or more additional openings.

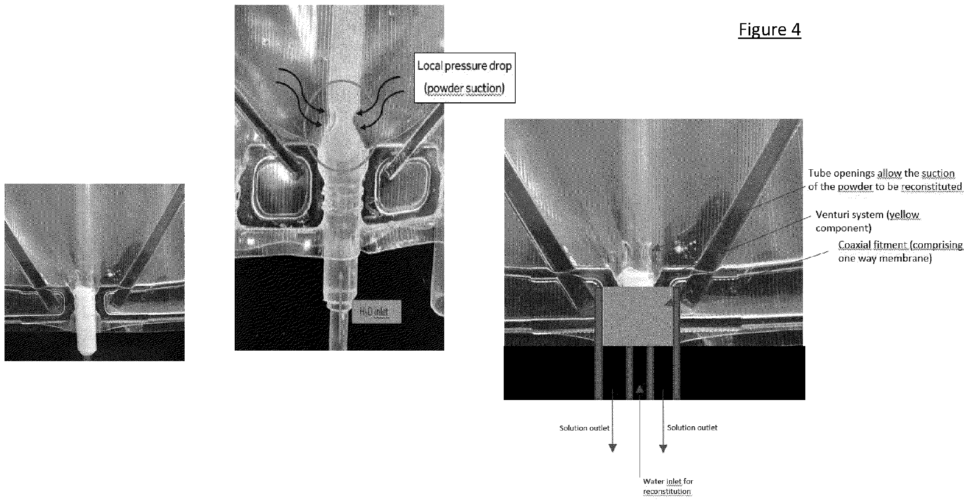

**Figure 4** provides photographs of a venturi nozzle of the invention in the first two panels (from left to right) and a photograph of a Venturi nozzle with a diagram of the outlet of the invention superimposed upon it (in the left hand panel).

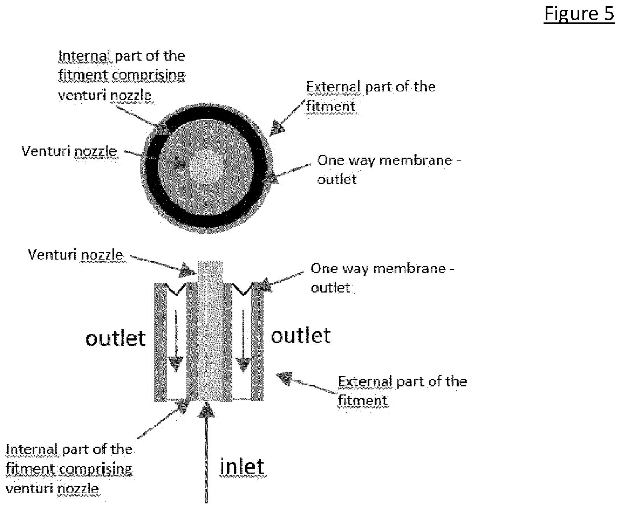

**Figure 5** provides a diagram of a fitment according to the invention. The upper panel shows a horizontal cross-section, wherein the central circle defines the venturi nozzle and the opening wherein fluid travels through the nozzle of the invention. The second inner-most ring of the upper panel represents the inner pipe of the outlet in cross-section. The black ring represents the one-way valve in the outlet, and the outermost ring represents the outer pipe of the outlet. The lower panel in **Figure 5** shows a transverse cross-section of the embodiment of the upper panel.

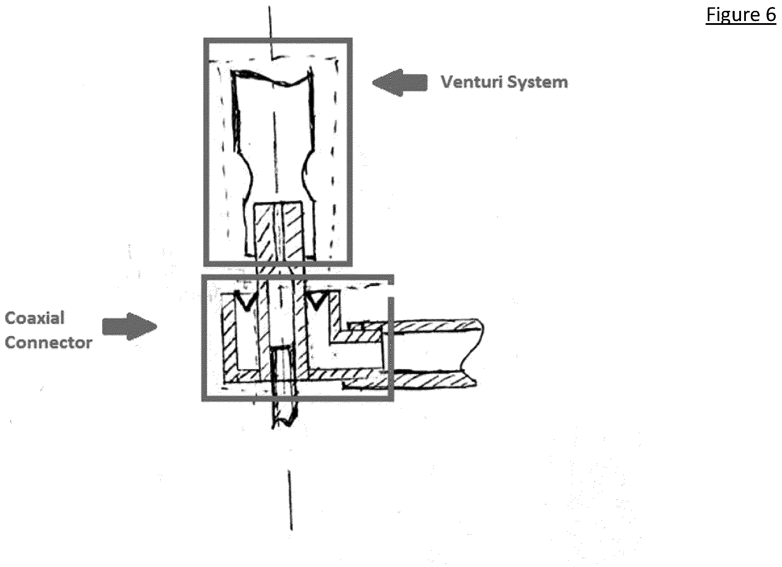

**Figure 6** illustrates an embodiment of the invention wherein the coaxial fitment comprises a coaxial connector, which interfaces directly with the fluid bag through an integral fitment and is fitted around the venturi nozzle.

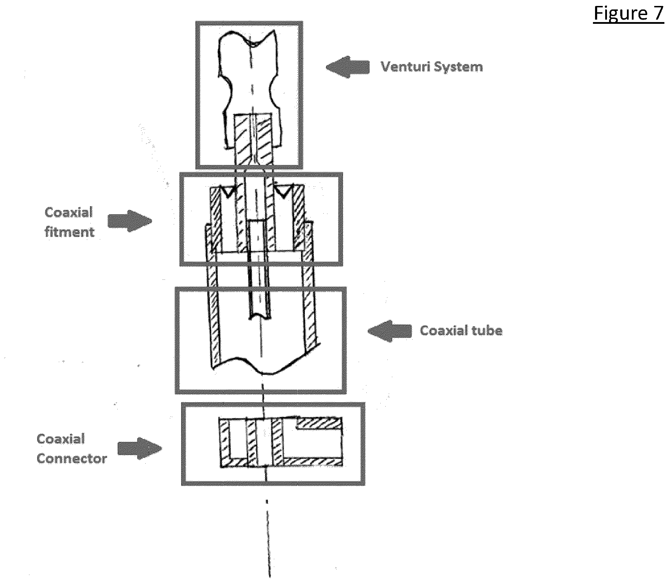

**Figure 7** illustrates an embodiment of the invention wherein the outlet comprises a coaxial fitment, which interfaces with the fluid bag and attaches a coaxial connector to the venturi nozzle. In this embodiment the coaxial fitment also comprises a coaxial tube which connects the fitment to the connector.

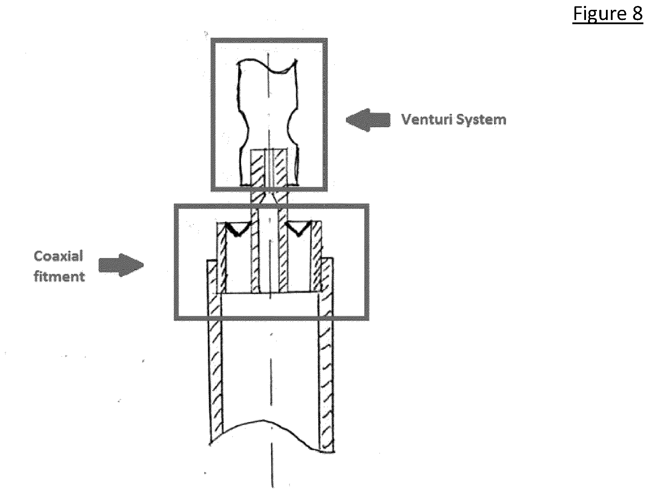

**Figure 8** illustrates an embodiment of the invention wherein the outlet is configured as a coaxial fitment. Said coaxial fitment is attached around the venturi nozzle to form an inlet/outlet system.

### DETAILED DESCRIPTION

### Definition

Unless defined otherwise, all technical and scientific terms used generally have the same meaning as commonly understood by one of ordinary skill in the art.

The articles "a" and "an" are used to refer to one or to over one (*i.e*., to at least one) of the grammatical object of the article. For example, "an element" means one element or over one element.

The term "adjacent" as used herein means next to or adjoining. In some embodiments, the venturi nozzle and outlet(s) of the invention are positioned adjacent each other in openings in the bag.

The term "approximately" as used herein means not exactly limited to. In particular, when used in relation to a direction, "approximately" means the direction may vary by several degrees to the central axis.

The term "at least one" encompasses one and more than one of the items thus described.

The term "bag" as used herein refers to a container, and preferably a flexible, collapsible and/or foldable container. Preferably a bag is a container for fluids, most preferably for liquids. The term "pouch" is used interchangeably with the term "bag".

The term "coaxial" or "co-axial", herein used interchangeably, refers to two items aligned with each other along an axis. Commonly this refers to two items "coaxially positioned", wherein their longitudinal axes are aligned. For example, the venturi nozzle and outlet of the invention. The alignment may be in 2-dimensional or in 3-dimensional space. Such an alignment forms a concentric arrangement, that is two features sharing the same centre, wherein the outer feature (for example the outlet of the present invention) completely surrounds the inner feature (for example the venturi nozzle of the present invention).

The term "comprising" includes, but is not limited to, whatever follows the word "comprising." Use of the term indicates the listed elements are required or mandatory but that other elements are optional and may be present.

The term "concentrated" as used herein means, of a substance or solution, present in a high proportion relative to other substances, preferably having had fluid, such as water or other diluting agents, removed or reduced.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, hemodialysis, hemofiltration, hemodiafiltration or ultrafiltration. A dialysis machine may be included within the flow path. A dialyzer may be included in the flow path.

The term "dissolution" as used herein refers to the process of a solute becoming incorporated into a solvent to make a solution. Dissolution means the same as "dissolving" and the terms are used interchangeably herein.

The term "dissolution time" as used herein refers to the time taken for one or more solutes to fully dissolve in a solvent to make solution. Dissolution times of the same solutes can be compared if the conditions of dissolution (including for example solvent type and volume, temperature, flow speed of solvent and container shape) are the same.

The term "distal" as used herein refers to the end of the venturi nozzle of the invention wherein the venturi nozzle is designed for the fluid to exit the venturi nozzle. This is the longitudinally most distant part of the venturi nozzle from fluid entry.

The term "duck valve", or "duckbill valve" refers to a one way valve comprising 2 or more flaps with a shape similar to the beak of a duck. It is used to prevent contamination due to backflow, particularly in medical applications. Said valves are preferably made from rubber or synthetic elastomer.

The term "equidistant" as used herein means at equal distances.

The term "filling time" as used herein means the time it takes for all the fluid being used as a solvent to be added to the solutes of a solution in a container (such as the bag of the invention). Filling time may be equal to dissolution time if the solutes dissolve quickly.

The term "fit" as used herein means to connect together without gaps.

The term "fitment" as used herein refers to a piece of equipment that it formed by several features fitting together to form one integrated and functional item. Specifcially it refers to an outlet of the present invention which is formed of inner and outer pipes or tubes, wherein a venturi nozzle of the invention fits into the hole within the centre of inner pipe or tune of the outlet with no gaps between the outer wall of the venturi nozzle and the inner wall of the outlet (as shown in Figure 5). The fit may be a snap fit. The venturi nozzle of the invention is configured to fit inside the outlet of the invention such that the section of the venturi nozzle comprising the lateral slots or holes in the sides protrudes beyond the outlet (and into the bag when fitted in the opening of said bag).

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid may be blood.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "formed" or "formed of" as used herein refers to a material comprising any of the materials, elements, composited or compounds listed.

The term "inlet" can refer to a component which fits into or attaches to an opening of another component, through which fluid and/or gas can be drawn into a component, conduit, or fluid passageway of any type, such as a bag, container, conduit, tube or pipe of any type. The inlets of the system described herein may comprise valves or nozzles. In preferred embodiments the inlets described here comprise tubing containing a one-way valve which prevents backflow of fluid back out of the inlet.

The term "internal diameter" as used herein refers to the diameter of the space within a product, and in particular the diameter of the space inside a tube, pipe, nozzle or other part of a flow path in which the fluid moves. The external diameter of such a product can be unrelated to the internal diameter. For example, the external diameter may be constant but the internal diameter, affecting the fluid flow, may change along the length of the product.

The term "measuring" or "to measure" can refer to determining any parameter or variable. The parameter or variable can relate to any state or value of a system, component, fluid, gas, or mixtures of one or more gases or fluids.

The term "measurement" refers to data or information of the parameter or variable determined.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The term "negative pressure" refers to pressure less than the surroundings. Commonly, this refers to pressure less than that of the pressure in the room where a venturi nozzle of the invention is being used.

The term "nozzle" as used herein refers to a fitting, usually at the end of a tube or pipe, which controls the flow of a fluid (liquid or gas) through said fitting. It may control (in speed, direction or otherwise) the entry of the fluid into a container, especially a bag. It may accelerate or decelerate the fluid. It may control the direction of the fluid. The nozzle may control the density of spray of the fluid and direction of spray of fluid from the nozzle. The nozzle may act as an inlet for fluid into a container. Generally, the term "nozzle" refers to a venturi nozzle.

The term "outlet" refers to a component which fits into or attaches to an opening in another component through which fluid or gas can be pulled out of said component and into another component, conduit, or fluid passageway of any type. The outlets of the system described herein comprise valves. In preferred embodiments the outlets described here comprise tubing containing a one-way valve which prevents backflow of fluid back into the initial component. In preferred embodiments the outlets of the present invention comprise an outer tube or pipe and an inner tube or pipe, with a ring-shaped one-way valve filling the gap between the inner and outer tubes or pipes. A nozzle of the invention fits into the inner tube or pipe to form a fitment of the present invention.

The term "opening" refers to gap, space or hole in a component through which fluid and/or gas can be drawn into or out of a component, conduit, or fluid passageway of any type, such as a bag, container, conduit, tube, nozzle or pipe of any type. An inlet or outlet, or a fitment of the invention, may fit into or attach to an opening.

The term "pass through" refers to an action of fluid or gas passing a component positioned in a flow path. The fluid or gas can enter an inlet of the component and exit via an outlet of the component to continue the flow path.

The term "pressure" refers to a force exerted by a gas or liquid on the walls of a component, container, or conduit.

The term "proximal" as used herein is used to refer to the end of the nozzle of the invention configured for fluid entry.

"Purified water" can be defined as water produced by distillation, deionization, reverse osmosis, or other suitable processes that remove impurities in water.

The term "reconstituting" or "re-constituting as used herein refers to restore to an original state. In the context of the solutions of the invention, this term is used to mean returning a solution to its original form, before liquids were removed, or in creating a solution from concentrated liquid and/or dried components.

The term "reconstituted" or "re-constituted" refers to an item, product or solution produced after reconstituting as defined above.

The term "ring-shaped" refers to circular shape with a central hole.

The term "solute" as used herein refers to a substance dissolved in a solution. The solute may comprise one or more chemicals or compounds. Solutes as used herein, before being dissolved in a solvent, may exist as entirely or partially dried, gels, concentrated liquids, pellets or powders, or any combination of these forms.

The term "solution" as used herein refers to a liquid mixture in which a solute is uniformly distributed within a solvent. A "solution for dialysis" may refer to any solution which can be used in any form of dialysis. It is envisaged that the solutions described herein contain specific concentrations of solutes.

The term "solvent" as used herein refers to a substance which dissolves a solute. Preferably the solvent is a liquid, and most preferably it is water.

The term "valve" as used herein refers to a device that regulates, directs or controls the flow of a fluid by opening, closing, or partially obstructing the path of said fluid. Valves may work automatically or may be controlled by a person or any external control. Preferably the values used herein are one-way valves, also known as check valves, clack valves, non-return valves, reflux valves, and retention valves. "One-way valves" are valves that normally allow fluid to flow through them in only one direction. A duck valve is an example of a one-way valve. As used herein, the "Venturi effect" reduction in fluid pressure, and increase in velocity, caused by a fluid flowing through a constricted section of a tube or pipe. Preferably the constriction is caused by a reduction in diameter of the tube or pipe. The valves used herein may comprise films or membranes.

The term "water purification unit" refers to any single component or system which can produce purified water (as defined above) from tap water or other water containing any type of impurity.

### System

The present invention concerns a system (10) comprising at least three parts, a bag (11), an outlet (15) and a venturi nozzle (12). The bag contains one or more solutes (13) in concentrated form, preferably in a powdered form. The venturi nozzle is for inserting fluid, preferably water, into the bag and thus re-constituting a solution by dissolving the solutes in the bag. The outlet (15) is in the form of an inner pipe surrounding an outer pipe, and the venturi nozzle (12) fits into the inner pipe leaving no gap between the outer wall of the venturi nozzle (12) and the inner pipe of the outlet (15). The outlet (15) and venturi nozzle (12) are thus coaxially aligned and form a fitment (17). The outlet further comprises a ring-shaped one-way valve (16) positioned in the space between the inner and outer pipes of the outlet.

The bag comprises at least one opening (14) wherein the fitment comprising the outlet and the venturi nozzle is configured to attach to said opening (either directly or via an attachment mechanism, port or fitting), so that the venturi nozzle can direct fluid into the bag and so that the outlet can direct fluid out of the bag. The fluid is prevented from returning to the bag by a one-way valve in the outlet.

Further features of the bag 11, outlet 15 and venturi nozzle 12 are described below.

### Nozzle

The venturi nozzle of the systems described herein comprises an internal diameter (D1; 21) which reduces along the length of the nozzle in order to create a venturi effect in fluid flowing through the nozzle.

The external diameter of the venturi nozzle of the invention may be consistent throughout the length of the nozzle, not reflecting the reduction in internal diameter inside. Alternatively, the external diameter of the venturi nozzle may reduce in line with the internal diameter. This may be step wise or tapered over the length of the nozzle. The external diameter may be any diameter, and may vary in any way, as long as it is wider than the internal diameter.

The venturi nozzle internal diameter (21) reduces in a gradient along the length of the nozzle from the proximal end. The gradient may be any angle. In preferred embodiment the gradient of the reduction in internal diameter may be at 5 degrees or less to the horizontal, at 10 degrees or less to the horizonal or at 20 degrees or less to the horizonal along the length of the nozzle (from the widest internal diameter to the narrowest).

The narrowest internal diameter of the venturi nozzle may have an internal diameter reduced by at least 5%, at least 10%, at least 15%, at least 20%, at least 25%, at least 30%, at least 35%, at least 40%, at least 45% or at least 50% compared to the end of the nozzle with the widest internal diameter. In some embodiments (such as for example, that shown in **Figure 1**) the distal end 22 of the venturi nozzle 12, where the fluid leaves the nozzle, has the widest point of internal diameter along the entire nozzle. Preferably the internal diameter of the venturi nozzle is increased by at least 5%, at least 10%, at least 15%, at least 20%, at least 25%, at least 30%, at least 35%, at least 40%, at least 45% or at least 50% compared to the point of narrowest internal diameter of the nozzle.

The venturi nozzle internal diameter may reduce in a step-wise manner along the length of the nozzle. The venturi nozzle preferably comprises at least, two, at least three, at least four, at least five or at least six or more sections (23), each with a reduced internal diameter compared to the proximally preceding section. Each section may have an internal diameter reduced by at least 5%, at least 10%, at least 15%, at least 20%, at least 25% or at least 30% compared to the preceding section.

The venturi nozzle of the invention may be made contiguously as one single part. Alternatively, it may be made from at least two, three, four, five, six or more subsections. These parts may correspond to sections with reduced internal diameters (D1, D2, D3).

At or around the narrowed internal diameter of the venturi nozzle there is at least one lateral hole or slot (30). As a result of the narrowing of the internal diameter of the venturi nozzle, fluid flowing through the venturi nozzle creates a negative pressure, that is pressure less than the surroundings, inside the venturi nozzle as the fluid passes the at least one lateral slot or hole. This creates a suction effect acting inwards through the at least one hole or slot. In other words, the negative pressure acts along the outside of the venturi nozzle, in the opposite direct to the fluid flow, from the distal end of the nozzle and into the at least one lateral hole or slot, wherein said lateral slot or hole is positioned at or around the narrowed point of the internal diameter (shown by arrows in **Figure 1** and **Figure 2**). The increasing of water inlet speed due to the consistent decreasing of the internal diameter, creates a local pressure drop that allows the generation of turbulent flow. In this specific case, the venturi effect is used to dissolve the solute, so the local pressure drop allows the powder to be forced through the holes. This increases dissolution rate and the generation of a turbulent flow that allows a homogeneous solution to be obtained.

The negative pressure forces air, and any components external to the venturi nozzle, such as fluids and or alternatively solutes, through the at least one lateral slot or hole of the nozzle, and to quickly exit the distal end of the nozzle, which has a wider internal diameter than the narrowest point of the internal diameter.

The negative pressure , and increase in velocity, in a fluid flowing through a constricted section of a tube or pipe, is known as the Venturi effect. Preferably fluid velocity is increased by at least 5%, at least 10%, at least 20% or at least 50%. A nozzle of the invention, which is configured to create the Venturi effect when fluid flows through it, may be known as a venturi nozzle. The systems of the invention may use any nozzle which can create a Venturi effect.

When released from the constriction inside the venturi nozzle, as the internal diameter of the nozzle increases towards the distal end of the nozzle, after the narrowest point of the internal diameter, the fluid may spread outwards. As the fluid, which at this point is a mixture of the fluid which entered the nozzle at the proximal end, along with any air, fluid or solutes which were dragged into the nozzle through the at least one lateral slot or hole due to the Venturi effect, leaves the distal end of the nozzle, it forms a spray due to pressure. The fluid is sprayed inside the bag of the invention. Preferably the spray is evenly distributed when leaving the nozzle. Preferably the spray contains small drops of even size.

The nozzle comprises lateral slots or holes 30. Preferably at least one, preferably at least 2, preferably at least 3 or more than 3 slots or holes. Preferably the slots or holes are equidistantly spaced from one another around the circumference of the nozzle. Said holes are placed distally to the narrowest part of the nozzle.

The venturi nozzle 12 may be made of any suitable material. Suitable materials include any plastic or polymer, or alternatively stainless steel, or a combination of any of these materials and/or other materials.

The venturi nozzle may comprise an external covering of any type, such as a pipe or tube 40. This may have any diameter greater than the largest external diameter of the nozzle. This external pipe may also comprise lateral slots or holes. Preferably at least one, preferably at least 2, preferably at least 3 or more than 3 slots or holes. Preferably the slots or holes are equidistantly spaced from one another around the circumference of the covering. In a preferred embodiment, the holes in the external covering align with the holes in the nozzle when the nozzle is inside the covering pipe or tube.

The venturi nozzle may comprise one or more coatings. Such coatings may be anti-stick or anti-friction coatings. Such coatings may be disinfectant, anti-microbial, anti-viral, or anti-coagulant. The coatings may be on the inside, outside or both sides of the nozzle. The inside and outside coatings may be the same or different. The external covering of the nozzle may also comprise one or more coatings, on the inside, outside or both. These coatings may be the same as on the nozzle, or different.

The venturi nozzle of the system described herein may simply fit into the centre of an outlet, forming a fitment wherein there is no gap between the outer wall of the nozzle and the inner wall of the outlet (the outlet comprising an inner pipe and an outer pipe, as described below). Alternatively, the fitment may be created integrally as piece comprising an inner Venturi nozzle of the invention surrounded by an outer outlet of the invention. The nozzle fits so that the section of the nozzle comprising the lateral slots or holes in the sides protrudes beyond the outlet (and into the bag when fitted in the opening of said bag).

The fitment may fit into the at least one opening 14 of the bag 11 of the system 10. Alternatively, it may be connected to the bag by a port, connector or other fixture to secure it to the bag and prevent leakage. It is envisaged that the widest part of the fitment, that is the widest external diameter of the outlet, fits in the at least one opening of the bag. The fitment may alternatively be welded to the bag or created integrally with the bag in one piece.

The connection of the fitment to the bag may be permanent, or it may be temporary and the fitment may be removable from the bag.

Preferably fluid is pumped through the venturi nozzle. The rate of flow of the fluid may be specific to the solution to be re-constituted.

Most preferably the fluid travelling through the nozzle is in a dialysis flow path. In preferable embodiments, the fluid is water, or purified water. The fluid may be heated, for example to body temperature or any other temperature that will increase the rate of dissolution of the solutes. The temperature of the fluid may be specific to the solution to be re-constituted.

### Bag

The system described herein contains a bag 11 comprising at least one opening 14. The bag should be leak-proof so that fluids cannot escape the bag.

The bag of the system described herein may be made of any material, preferably a polymer, most preferably a plastic. The bag may be rigid or flexible. Preferably the bag is flexible, resistant and stretchable, most preferably in any direction. The bag may be made of a multilayered material, or a single layered material.

In one embodiment the bag of the system described herein contains a v-shaped, funnel shaped or cone shaped section, with an opening for fitment of the invention (discussed below) at the point of the cone. Said opening should be placed at the lowest point of the bag when the system is in use. Such a shape maximises the mixing of the fluid spray leaving the nozzle with the solutes in the bag. **Figure 3** shows the direction of the longitudinal axis (L) of the nozzle and of the outlet shown in that figure.

The bag may further comprise additional openings for outlets or inlets.

In one embodiment, as shown in **Figure 3**, the bag comprises two openings. A central opening for the fitment at the bottom, and additionally the top of the bag is open.

The bag described herein can be replaced by a solid container, preferably shaped with a cone-shaped part in which to fit a venturi nozzle as described herein. Said container may be a box or carton, and is preferably made of a plastic. Said container may comprise a cone-shaped part made of a different, more flexible material. Alternatively, a flexible bag may comprise a rigid cone-shaped part for attachment to the nozzle.

In preferred embodiments the bags of the invention have a small volume, such as 3I or less, 2I or less, 1.51 or less, 1I or less, or 0,51 or less.

The bag contains one or more solutes in concentrated form. The solutes may be mixed together in this concentrated form, or they may be separate. The solutes may be separated by packaging, preferably by dissolvable packaging.

Examples of solutes may for example comprise the following or any combination thereof:- all electrolytes, powdered acids and bases, citric acid, sodium chloride, hydrogen carbonate, calcium and magnesium ions and salts, sodium, potassium, citric acid, sodium bicarbonate and all bicarbonate salt forms, sodium lactate, and osmotic agents such as glucose, dextrose, polydextrose, polydextrine and xylitol. Dissolution of the solutes may create a medically useful solution, such as a dialysate.

The bag may comprise one or more coatings. Such coatings may be anti-stick or anti-friction coatings. Such coatings may be disinfectant, anti-microbial, anti-viral, or anti-coagulant. The coatings may be on the inside, outside or both sides of the bag. The inside and outside coatings may be the same or different.

The bag may comprise multiple additional openings. In some embodiments further nozzles of the invention or of different types, may attach to such openings. Additional openings may allow the removal or addition or further components. The additional openings may be connected to a dialysis flow path, via outlets or inlets.

### Outlet

The system described herein comprises an outlet. Said outlet comprises an external pipe or tube which fits into or attaches to an opening in the bag, a narrower inner pipe or tube which fits inside the external pipe or tube to form an annular fitment which surrounds the venturi nozzle, and a ring-shaped one-way valve configures to fit in the space between the two tubes. The purpose of the outlet is to remove the re-constituted fluid from the bag.

The outlet of the invention comprises a one-way valve between the two concentric pipes or tubes (as shown in cross section in Figure 5). In preferred embodiments this is a ring-shaped one-way valve. In use, the one-way valve allows fluid the leave the bag only. That is, the one-way valve operates in the opposite direction to the venturi nozzle of the invention, said nozzle directing fluid into the bag only. The valve may comprise a film or membrane. In one embodiment the one-way valve is a duck valve. It is envisioned that valves used herein work automatically and are not controlled by a person or any external control. However, a manually user controlled or electronically controlled valve may be used in some embodiments.

The venturi nozzle of the invention should be placed coaxially into the centre of the outlet (i.e. inside the narrower inner pipe or tube), next to the one-way valve (i.e. at the same end of the outlet as the one-way valve). The fitment formed by the coaxial position of the nozzle and the outlet should be placed in an opening at or close to the lowest point of the bag when the system is in use. This enables the solution to drain through the at least one outlet via gravity. Alternatively or in addition, the outlet may be placed under suction.

When the fitment is attached to the bag, the venturi nozzle is positioned to direct fluids into the bag and the one-way valve of the outlet is positioned to direct fluids out of the bag. i.e. the one way valve prevents back flow into the bag.

**Figure 3** shows the direction of the longitudinal axis (L) of the fitment.

The outlet of the invention may be connected to a dialysis flow path.

The flow path of the fluid can be divided after it passes through the outlet. The flow path may be a dialysis flow path.

The bag of the invention may comprise one or more additional opening, and said openings may comprise one or more additional outlets or any type. Said openings and outlets may remove components from the bag if necessary, for example excess gas.

In one embodiment, the outlet comprises a fitment which comprises an annular one-way valve surrounding the venturi nozzle, and an attachment for a tube to remove fluid from the bag, for example at right angles to the axis of the venturi nozzle.

In another embodiment, the outlet comprises a fitment which comprises an annular one-way valve surrounding the venturi nozzle, and a coaxial connector which is connected to the fitment by means of a coaxial tube, or directly. The coaxial connector comprises an attachment for a tube to remove fluid from the bag.

In a further embodiment, the outlet comprises a fitment which comprises an annular one-way valve surrounding the venturi nozzle, and connects to a coaxial tube which provides fluid to the venturi nozzle and removes fluid from the outlet. Thus, fluid enters and leaves the bag by the same tube.

### Kit

The bags and nozzles disclosed herein can be provided in a kit.

Said kits may comprise at least a bag 11, an outlet 15, and a nozzle 12, the bag of the invention comprising one or more solutes which can be dissolved in fluid, and an opening. The outlet comprises and inner pipe surrounding an outer pipe. The nozzle configured to fit into the inner pipe of the outlet, forming a fitment (17). The outlet comprises a ring-shaped one-way valve between the inner pipe and the outer pipe, said one-way valve surrounding the central nozzle in the fitment. The fitments fits withing the opening of the bag. The one-way valve is orientated to operate in the opposite direction to the nozzle of the invention, said nozzle directing fluid into the bag only, said outlet allowing fluid to leave the bag only.

Said kits may further comprise connectors and/or tubing/pipping.

Said kits may also comprise a water purification unit.

Said kits may also include one or more tags or labels, such as smart tags, to enable identification of the parts and bags, and specifically the contents of the bags.

### Methods

Also described herein are methods of use of the bags connectors and and venturi nozzles disclosed.

A method of re-constituting a solution comprising one or more solutes in a fluid is described herein. Said method comprises the step of:-
flowing a fluid through a venturi nozzle, said nozzle comprising an internal diameter which reduces along the length of the nozzle in order to create a venturi effect in fluid flowing through the nozzle; and said nozzle comprises at least one lateral slot or hole at or around the narrowest part of the nozzle, and wherein the internal diameter of said nozzle widens distally from the at least one lateral slot or hole in order to create a venturi effect in fluid flowing through the nozzle;
wherein the distal end of the nozzle is positioned facing into an opening in a bag containing one or more solutes in concentrated form,
such that the venturi effect created in the fluid flowing into the bag improves the mixing of the fluid and the one or more solutes;and
allowing the re-constituted solution to leave the bag via an outlet comprising a one-way valve, said outlet being positioned coaxially to the nozzle along the longitudinal axis, said nozzle fitting inside said outlet to form a fitment, said one-way valve being ring-shape around the central nozzle in said fitment, said fitment fitting to the opening of the bag.

Additional steps may include one or more of the following:-
Heating the fluid to an appropriate temperature to improve dissolution before said fluid enters the nozzle;
Controlling the fluid flow rate into the nozzle;
Selecting the solutes in the bag to determine the type of the re-constituted solution; and/or
Selecting the volume of fluid that flows into the bag to determine the concentration of the re- constituted solution.

Furthermore, the method may include the step of connecting the bag to a dialysis flow path, such that the re-constituted solution may circulate in the dialysis flow path. Specifically, the outlet may be connected to a dialysis flow path.

Also described herein is a system for dialysis wherein a bag and fitment as described herein are fluidly connected to a dialysis machine of any type. The solutes in the bag may be dissolved in fluid before or during the treatment. A patient may be fluidly connected to the bag and fitment and the dialysis machine. More than one bag and fitment of the current invention may be fluidly connected to the system. The system may comprise further components.

Also described herein is a method of dialysis comprising the steps of:-
Fluidly connecting a system comprising a bag, an outlet and a nozzle of the current invention to a dialysis machine; and
fluidly connecting said dialysis machine and bag and nozzle to a patient.
A step of adding fluid to the bag through the nozzle and creating or reconstituting a solution in the bag may be performed before or after fluidly connecting the bag and fitment to a dialysis machine, or before or after fluidly connecting the bag and fitment to a patient.

A further step of running the dialysis is then carried out.

More than one bag and fitment of the current invention may be fluidly connected to the dialysis machine and to the patient.

Also described herein is a method of dialysis comprising the steps of:-
using the system described herein to re-constitute a solution, and using said solution in dialysis.
Said method may further comprise the step of fluidly connecting a system of the current invention to a dialysis machine; and
fluidly connecting said system to a patient.

The method of dialysis may comprise an initial priming step, wherein the dialysis machine is primed before connection to the patient. The bag, outlet and nozzle of the invention may be connected before or after this step. The solution in the bag may be created or reconstituted before or after the priming step.

### Uses

The systems and kits of the invention may be used in dialysis, particularly hemodialysis peritoneal dialysis, hemofiltration, hemodiafiltration or ultrafiltration. Particularly they are used in dialysis carried out at home, or another environment away from a hospital or medical practice. They may also be used in a hospital or any kind of medical practice.

Use of the bags, outlets, venturi nozzles or fitments comprising a nozzle and an outlet, of the invention may also occur in the process of sterilizing a dialysis machine and all associated equipment in a dialysis flow path.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. A system (10) for re-constituting a solution comprising one or more solutes in a fluid, said system comprising a bag (11) containing one or more solutes in concentrated form, a nozzle (12) for inserting the fluid into the bag, and an outlet (15), wherein:-
said outlet comprises a ring-shaped one-way valve (16) allowing fluid to leave the bag only,
wherein said nozzle is configured to fit inside the outlet in a coaxial position along the longitudinal axis (L), forming a fitment,
wherein the fitment is configured to fit or attach to an opening (14) in the bag such that the nozzle is positioned to direct fluids into the bag and the one-way valve of the outlet is positioned to direct fluids out of the bag;
wherein said nozzle comprises an internal diameter which reduces along the length of the nozzle, and said nozzle comprises at least one lateral slot or hole (30) at or around the narrowest part of the nozzle, and wherein the internal diameter of said nozzle widens distal to the at least one lateral slot or hole (30) in order to create a venturi effect in fluid flowing through the nozzle;
such that the venturi effect created in the fluid flowing through the nozzle and into the bag improves the mixing of the fluid and the one or more solutes.

2. The system according to claim 1 wherein the nozzle internal diameter reduces in a gradient along the length of the nozzle or wherein the nozzle internal diameter reduces in a step-wise manner along the length of the nozzle.

3. The system according to any preceding claim, wherein the bag comprises a V or cone shaped end, wherein the opening for the fitment is positioned at the point of the V or cone.

4. The system according to any preceding claim, wherein the one or more solutes are reconstituted into a solution for dialysis.

5. The system according to claim 4 wherein said dialysis is peritoneal dialysis, hemodialysis, hemofiltration, hemodiafiltration or ultrafiltration.

6. The system according to any preceding claim, wherein the one or more solutes are reconstituted into a dialysate.

7. The system according to any preceding claim, further comprising a water purification unit configured to be fluidly connectable to the nozzle.

8. The system according to any preceding claim, wherein the outlet is fluidly connectable to a dialysis flow path.

9. The system according to any preceding claim, wherein the nozzle comprises at least 2, preferably at least 3 or more than 3 slots or holes, preferably wherein the slots or holes are equidistantly spaced from one another around the circumference of the nozzle at or around the narrowest internal diameter of the nozzle.

10. The system according to any preceding claim, wherein the nozzle comprises an external covering having any diameter greater than the largest external diameter of the nozzle.

11. The system according to claim 10 wherein the external covering comprises at least one lateral slots or hole, preferably at least 2, preferably at least 3 or more than 3 slots or holes, preferably wherein the slots or holes are equidistantly spaced from one another around the circumference of the covering, most preferably wherein the holes in the external covering align with the holes in the nozzle when the nozzle is inside the covering.

12. The system according to claims 1-12 for use in dialysis.

13. A kit for use in dialysis, said kit comprising:-
a nozzle wherein the internal diameter of the nozzle reduces along the length of the nozzle, and said nozzle comprises at least one lateral slot or hole, in order to create the venturi effect in fluid flowing through the nozzle;
a bag containing one or more solutes which can be dissolved in a fluid, said bag comprising at one opening;
at least one outlet comprising a one-way valve;
wherein the nozzle is configured to fit into a central opening of the outlet, coaxial to the outlet, forming a fitment (17), wherein the one-way valve is ring-shaped and surrounds the central nozzle in the fitment, and wherein the fitments fits within the opening of the bag.

14. The kit according to claim 13, further comprising a water purification unit.

15. A method of re-constituting a solution, comprising the steps of:-
(i) filling a bag comprising solute constituents of the solution with fluid passing through a nozzle, wherein the internal diameter of said nozzle reduces along the length of the nozzle, and said nozzle comprises at least one lateral slot or hole, in order to create the venturi effect in fluid flowing through the nozzle; and
(ii) allowing the liquid contents of the bag to leave the bag via an outlet comprising a one-way valve, said out being positioned coaxially to the nozzle along the longitudinal axis (L).
